# EUROPEAN PATENT SPECIFICATION

(11) **EP 0 273 660 B1**
(45) Date of publication and mention of the grant of the patent: **28.07.1993**
(21) Application number: 87311199.1
(22) Date of filing: 18.12.1987
(51) Int. Cl.: C12N 15/09, C12P 19/40, C12N 1/20, C12N 15/90

(54) **DNA encoding an inactivated IMP dehydrogenase**
Für inaktivierte IMP-Dehydrogenase kodierende DNS
ADN codant pour une IMP-déshydrogénase inactivée

(30) Priority: 26.12.1986 JP 311588/86
(43) Date of publication of application: 06.07.1988
(73) Proprietor: Takeda Chemical Industries, Ltd., Chuo-ku, Osaka (JP)
(72) Inventor: Miyagawa, Kenichiro, Toyono-gun Osaka 563-01 (JP); Kimura, Hiroyuki, Sakai Osaka 590 (JP); Sumino, Yasuhiro, Suma-ku Kobe Hyogo 654 (JP)
(74) Representative: Laredo, Jack Joseph

(56) References cited:
- EP-A- 0 074 808
- EP-A- 0 127 328
- EP-A- 0 151 341
- WO-A-86/01825
- BIOTECHNOLOGY, vol. 4, March 1986, pages 225-228; K. MIYAGAWA et al.: "Cloning of the Bacillus subtilis IMP dehydrogenase gene and its application to increased production of guanosine"
- PROC. NATL. ACAD. SCI. USA, vol. 80, August 1983, pages 4894-4898; N.I. GUTTERSON et al.: "Replacement and amplification of bacterial genes with sequences altered in vitro"
- CHEMICAL ABSTRACTS, vol. 106, no. 17, 27th April 1987, page 191, no. 132978s, Columbus, Ohio, US; S.R. FAHNESTOCK et al.: "Protease-deficient Bacillus subtilis host strains for production of staphylococcal protein A", & APPL. ENVIRON. MICROBIOL. 1987, 53(2), 379-84
- Agric. Biol. Chem., vol. 46(9), pages 2347-2352, 1982

## Description

The present invention relates to a production method for inosine, an important substance as a starting material for the synthesis of 5ʹ-inosinic acid, novel microbes used for the production thereof and novel DNAs used for the construction of said novel microbes.

As to the production of inosine by fermentation, methods are known which use a *Bacillus* strain (Japanese Published Examined Patent Application No. 2956/1980, Japanese Published Examined Patent Application No. 41915/1982, Japanese Published Unexamined Patent Application No. 42895/1984), *Brevibacterium* strain [Japanese Published Examined Patent Application No. 5075/1976, Agricultural Biological Chemistry, *42*, 399 (1978)] or the like having either an adenine requirement or various drug resistances as well as an adenine requirement. These are thought of as bacterial strains whose purine nucleotide biosynthesis system has been strengthened.

On the other hand, obtaining IMP dehydrogenase deficient strains is thought of as useful in a method of mass accumulation of inosine because it is expected that when IMP dehydrogenase (5ʹ-inosinic acid dehydrogenase) is defective, 5ʹ-inosinic acid, an intermediate for inosine production, efficiently converts itself to inosine without converting itself to 5ʹ-guanylic acid. For this purpose, xanthine- or guanine-requiring strains have conventionally been selected using the replica method or the like via mutagenic treatments such as ultraviolet irradiation and nitrosoguanidine (NTG) treatment.

However, auxotrophs thus obtained have presented some drawbacks; for example, their productivity is in some cases lower than that of their parent strains due to mutations in regions on the chromosome other than the relevant enzyme gene, and their productivity often fluctuates decreases due to back mutation during fermentation.

In the light of these circumstances the present inventors made investigations of how to obtain IMP dehydrogenase deficient strains; as a result, the present inventors succeeded in constructing novel microbes having a chromosomal DNA of the structure in that an IMP dehydrogenase-encoding gene DNA incorporates a suitable DNA fragment selected from the outside of said gene DNA using recombinant DNA techniques. The present inventors found that said novel microbes induced using said techniques from recipient microbes selected out of adenine-requiring *Bacillus* bacteria can be repeatedly used for the highly stable production of inosine since they accumulate a large amount of inosine and the back mutation rate for IMP dehydrogenase activity during fermentation is not more than the detection limit. The present invention was accomplished based on this finding.

That is, the present invention involves:
1. A DNA encoding an inactivated IMP dehydrogenase, which is highly homologus to that of an IMP dehydrogenase gene region of *Bacillus* strains and/or its flanking regions.
2. The DNA as defined in the item (1) above, wherein said DNA has an IMP dehydrogenase gene region inserted a DNA fragment to render said gene inactivated.
3. The DNA as defined in the item (2) above, wherein the DNA fragment is a gene other than an IMP dehydrogenase gene.
4. The DNA as defined in the item (3) above, wherein the other gene is a selection marker gene.
5. The DNA as defined in the item (1) above, wherein said DNA is one which is deleted a part or the whole of IMP dehydrogenase gene region.
6. Vectors containing a DNA as defined in the item (1), (2), (3), (4) or (5) above.
7. A *Bacillus* strain transformed with the DNA as defined in the item (1) to (5) above or vectors as defined in the item (6) above, and
8. A method of producing inosine which comprises cultivating in a medium an IMP dehydrogenase deficient *Bacillus* strain which has been transofrmed with a DNA encoding an inactivated IMP dehydrogenase, which is highly homologus to that of an IMP dehydrogenase gene region and/or its flanking regions, or a vector with said DNA inserted therein and which is capable of producing inosine, and harvesting inosine so produced in the culture medium.

For the DNA containing an IMP dehydrogenase gene region for the present invention, a DNA cut off from microbial chromosomal DNA by a conventional method using a restriction enzyme can be used. For example, a DNA isolated and cloned by the method described in Japanese Published Unexamined Patent Application No. 156388/1985 and the corresponding European Patent Publication No. 0151341 can be used. In this case, it is desirable that the DNA contain both the entire region of an IMP dehydrogenase-encoding DNA and a few flanking regions before and behind thereof; however, DNAs containing either a partof the region of an IMP dehydrogenase gene or both a part of the region of said gene and flanking regions thereof may be used.

In the above definition, any DNA can be used for the entire region of an IMP dehydrogenase-encoding DNA, as long as it has a 1.85 kilobase pairs of *Xba* I*-Hin* CII fragment.

For the donor of an IMP dehydrogenase gene, any microbe can be used in principle without any special limitation on its origin, as long as the base sequence of the IMP dehydrogenase gene of the relevant bacterial strain is highly homologous to that of the IMP dehydrogenase gene on the chromosome of the *Bacillus* strain used as a recipient as described below. Specifically, the use of a *Bacillus* strain is preferable also from the point of view of handling because of what is called self-cloning; in addition, the transformant thus obtained is expected to be stable. It is not always necessary for said donor to have a capability of producing inosine, guanosine, xanthosine or their purine bases, but the use of a microorganism which is not deficient in IMP dehydrogenase is preferable. As examples of such DNA donors, mention may be made of *Bacillus* strains such as *Bacillus subtilis, Bacillus pumilus* and *Bacillus licheniformis*. Bacterial strains which can be used include the following known microorganisms:
*Bacillus subtilis* NA-6011(IFO 14189, FERM BP-291)
*Bacillus subtilis* NA-6012(IFO 14190, FERM BP-292)
*Bacillus subtilis* NA-7821(IFO 14368, FERM BP-618)
*Bacillus subtilis* NA-6128(IFO 14373, FERM BP-617)
*Bacillus subtilis* No. 115 (IFO 14187)
*Bacillus subtilis* No. 168 (BGSC No. 1A1)
*Bacillus subtilis* MI 114 [Gene,*24*,255(1983)]
*Bacillus pumilus* (FERM P-2116)
*Bacillus pumilus* (FERM P-4832)
*Bacillus pumilus* (FERM P-4829)
*Bacillus licheniformis* ATCC 8480
*Bacillus licheniformis* IFO 12485
- BGSC: ; The Bacillus Genetic Stock Center
- IFO: ; Accession number at Institute for Fermentation, Osaka (IFO), 17-85 Juso-honmachi 2 chome Yodogawa-ku, Osaka 532, Japan
- FERM P: ; Accession number at Fermentation Research Institute (FRI), Agency of Industrial Science and Technology, Ministry of International Trade and Industry, 1-3, Higashi 1-chome, Yatabemachi, Tsukuba-gun, Ibaragi-ken, Japan
- FERM BP: ; FRI accession number based on the Budapest Treaty.

In addition, already cloned IMP dehydrogenase genes of a *Bacillus* strain can certainly also be used as donors of DNA in the present invention, whatever host-vector system has been used. As an example of such a cloned IMP dehydrogenase gene, mention may be made of pEX 117, resulting from the cloning of an IMP dehydrogenase gene-containing DNA derived from *Bacillus subtilis* strain NA-6012 onto pBR 322 (Japanese Published Unexamined Patent Application No. 156388/1985).

pEX 117 can be extracted from *Esherichia coli* TEX 117 carrying the said plasmid by the method described in "Molecular Cloning, A Laboratory Manual", T. Maniatis et al., Cold Spring Harbor Laboratory, published by University of Tokyo Press, 1982, pp. 86 to 93.

For the construction and isolation of the DNAs of the present invention, the use of recombinant DNA techniques is convenient. For the host-vector system used for this purpose, EK systems can be suitably used, i.e., strains which can be suitably used as hosts include strains derived from *Esherichia coli* strain K-12 such as *Esherichia coli* C600 ("Molecular cloning", p. 504), *Esherichia coli* JA221 (IFO 14359) and *Esherichia coli* 294 [Proc. Natl. Acad. Sci. USA, *73*, 4174 (1976)] and strains derived therefrom [e.g. *Esherichia coli* X-895 (IFO 14308, FERM BP-614) induced from said C600 strain], and vectors which can be suitably used include pBR 322, pACYC 184, pACYC 177, pUC18, pUC19, pHSG 396 and pHSG 298.

The novel DNAs of the present invention can be constructed and isolated from plasmids containing an IMP dehydrogenase gene using the procedure shown below.

The desired DNA can be obtained by digesting a plasmid containing an IMP dehydrogenase gene with a restriction enzyme by a conventional method, separately cutting the DNA fragment to be inserted with restriction enzymes by a conventional method, extracting and isolating the said cutting after separation by for example agarose gel electrophoresis if necessary, mixing both together, and ligating them together using T4 DNA ligase either directly in cases where their cohesive ends are the same with each other or after converting their cohesive ends to blunt ends using T4 DNA polymerase in cases where the cohesive ends are different from each other.

For the restriction enzyme for digesting a plasmid containing an IMP dehydrogenase gene, any restriction enzyme can be used, as long as it has some restriction sites on the relevant gene DNA; however, it is preferably that a restriction enzyme which has a small number of restriction sites on the relevant gene and which does not digest any other region of the relevant plasmid be selected. For example, *Afl* II*, Apa* I*, Axy* I*, Bam* HI*, Bcl* I*, Bgl* II*, Bst* EII*, Cla* I*, Eco* RI*, Eco* 81I*, Hpa* I*, Hin* dIII*, Kpn* I*, Mlu* I*, Nco* I*, Pst* I*, Sac* I*, Sac* II*, Sal* I*, Sma* I*, Sph* I*, Spl* I*, Ssp* I*, Stu* I*, Xba* I and *Xho* I can be suitably used.

In the present invention, for the DNA fragment to be inserted into IMP dehydrogenase gene region, any DNA fragment can be used of whatever origin it is, as long as it renders said gene inactivated, including DNA fragments of procaryotes and eucaryotes. Normally, it is advantageous for practical application to use a DNA fragment containing a selection marker gene so that IMP dehydrogenase is made inactivated and transformant selection becomes easier.

Any gene can be used as such as selection marker, as long as it expresses itself in a *Bacillus* strain, but it is preferable that the marker gene express itself in *Escherichia coli*. In this case, marker gene expression in a *Bacillus* strain and that in *Escherichia coli* may be of the "read-through" type; it is therefore not always necessary for the DNA fragment to be inserted to have a promoter expressing itself in the relevant host.

As examples of such marker genes, mention may be made of drug resistance genes, amino acid biosynthesis genes, nucleic acid biosynthesis genes and vitamin biosynthesis genes. As examples of drug resistance genes, mention may be made of *Bacillus pumilus* chloramphenicol acetyltransferase gene [D.M. Williams et al.; J. Bacteriol., *146*, 1162 (1981)], plasmid pC 194 chloramphenicol acetyltransferase gene [S. Horinouchi and B. Weisblum; J. Bacteriol., *150*, 815 (1982)] and plasmid pUB 110 kanamycin mucleotidyltransferase gene [M. Matsumura et al.; J. Bacteriol.; *160*, 413 (1984)]. As examples of amino acid biosynthesis genes, mention may be made of *Bacillus subtilis* leucine gene [T. Tanaka and K. Sakaguchi; Molec. Gen. Genet., 165, 269 (1978)] and *Bacillus amyloliquifacience* tryptophan gene [K. Yoshimura et al.; J. Bacteriol., *159*, 905 (1984)]; as an example of nucleic acid gene, mention may be made of *Esherichia coli* thymidine synthetase gene [Rubin et al.; Gene, *10*, 227 (1980)]; as an example of vitamin gene, mention may be made of mouse dihydrofolate reductase [D.M. Williams et al.; Gene, *16*, 199 (1981)].

Transformants having an inserted and inactivated IMP dehydrogenase gene can be easily selected either by adding the relevant antibiotic to the medium for selection in cases where, for example, a DNA containing a drug resistance gene is used or by complementing auxotrophy in cases where a biosynthesis gene is used as a marker.

The construction of a selection marker gene DNA fragment can be achieved as directed below.

A selection marker gene DNA can be isolated from a small piece of agarose gel containing said gene DNA in accordance with a published method such as the method described in Molecular Cloning, pp. 164 to 166 after fractionating by agarose gel electrophoresis fragments obtained by digesting a recombinant containing said gene with a suitably selected restriction enzyme. For the restriction enzyme used in this case, a restriction enzyme which has no restriction site on the relevant gene and which provides as small a fragment containing said gene as possible is preferably selected. Two suitable restriction enzymes may be used in combination as occasion needs. In cases where either end of the selection marker gene DNA fragment thus obtained does not fit the cohesive end of the restriction site of the IMP dehydrogenase gene into which said DNA fragment is to be inserted, ligation is made possible by converting said cohesive end to a blunt end.

On the other hand, in the present invention the DNA which deletes a part or the whole of IMP dehydrogenase gene region and has flanking regions of said gene can be obtained as follows:
The plasmid containing the IMP dehydrogenase gene is digested completely or partially with the restriction engyme whose cleavage site(s) is/are on the relevant gene region. The desired DNA is obtained by religating the above digested DNA after its cohesive ends was converted to blunt ends. Said flanking regions include DNAs having about 0.05 kilo base pairs or more, preferably about 0.1 to 10 kilo base pairs apart from the respective 5ʹ-upstream and 3ʹ-downstream of IMP dehydrogenase gene region.

The procedure to obtain the DNA of the present invention is further explained in detail, using a restriction enzyme having two cleavage sites on the relevant IMP dehydrogenase gene region for example.

The recombinant plasmid containing the relevant gene is digested with the restriction enzyme, and then said enzyme is inactivated by a procedure such as heating, for example. The DNA is purified with phenol, precipitated with cold ethanol and religated with a T4 DNA ligase.

Another procedure may be also adapted: after digestion with the restriction enzyme, the DNA fragment to be deleted is removed by using agarose gel electrophoresis from the digested product and then the remaining DNAs are ligated with a T4 DNA ligase. Thus, the above methods give the DNA of the present invention which deletes a small fragment according to two cleavage sites of the restriction enzyme on the DNA region encoding IMP dehydrogenase.

In the case where the DNA according to the present invention is prepared using a restriction enzyme has three or more cleavage sites on the IMP dehydrogenase gene region or at least one cleavage site on some region of the plasmid containing IMP dehydrogenase gene region in addition to cleavage site(s) on the relevant gene region, the desired DNA may be obtained by religating after partial digestion of said plasmid DNA with said restriction enzyme. Further, the DNA of the present invention may be also obtained using a restriction enzyme which has the sole cleavage site on the IMP dehydrogenase gene region. In this case, the starting DNA is digested with such a restriction enzyme, and the resulting digested DNA, after their cohesive ends, if arose, were converted to blunt ends, is religated to give the desired DNA. Addition of a nucleotide to cohensive ends or removal of a nucleotide from cohensive ends occurs on the occasion of conversion, and the desired DNA can be obtained. Deletion of a fragment from the IMP dehydrogenase gene region is also achieved using a couple of different kinds of restriction enzymes, each of which has individual cleavage site on the gene region.

A novel DNA of the present invention can be obtained in a large amount by transforming the host strain with a T4 DNA ligase ligation mixtures as described above to obtain a transformant growing on a medium for selection corresponding to the inserted selection marker, and proceeding with this transformant in accordance with a *per se* known method such as the above-mentioned method described in "Molecular Cloning", pp. 86 to 93.

As to the isolation of the marker-inserted IMP dehydrogenase gene region from the plasmid thus obtained, said gene region can be easily extracted and isolated using, for example, an electroeluter after separating by agarose gel electrophoresis fragments obtained by cleaving said plasmid with a restriction enzyme by a conventional method.

On the other hand, a DNA which deletes a part or the whole of IMP dehydrogenase gene can be obtained in a large amount by transforming the xanthine requiring host strain with a T4 DNA ligase ligation mixture as described above to obtain a transformant which is capable of growing on a minimum medium to which a substance essential for growth of said host strain and a selection drug are added and is uncapable of growing on a medium containing no xanthine.

The method of constructing a novel microbe by transforming a *Bacillus* strain with the novel DNA thus obtained is described below.

Any microorganism can be used as a recipient for this transformation, as long as it has an ability to take up a linear DNA provided outside the bacterial cell followed by recombination in a region highly homologous to the base sequence of said DNA on the chromosome to transform itself, namely, what is called capability of transformation; it is not always necessary for the recipient to be identical with the IMP dehydrogenase gene donor, as long as the base sequence of its chromosomal DNA exhibits a high homology to that of the IMP dehydrogenase gene contained in the linear DNA. For the purpose of obtaining transformants possessing high inosine productivity, *Bacillus* strains, specifically *Bacillus subtilis, Bacillus pumilus, Bacillus licheniformis* and the like are preferable from the point of view of the fact that these microbes are known to accumulate at least 1 of the purine substances such as inosine, guanosine and xanthosine, they possess a capability of transformation, they have no pathogenicity, and they have been safely used also in industrial situations.

In addition, when one of these microbes, previously provided with either 1 or not less than 2 of the known characteristics which are thought of as favorable for the accumulation of purine substances, such as purine analogue resistances including 8-azaguanine resistance, nucleoside phospholylase deficiency and folate antagonist resistance, is used as a recipient, more favorable results for the improvement of inosine productivity are obtained in many cases.

As examples of *Bacillus* bacteria which can be used as recipients, mention may be made of the following:
*Bacillus subtilis* (B.G.S.C. No. 1A3)
*Bacillus subtilis* BM 1032 (IFO 14559, FERM BP-1242; deposited on December 25, 1986)
*Bacillus subtilis* NA-6011 (IFO 14189, FERM BP-291)
*Bacillus subtilis* NA-6012 (IFO 14190, FERM BP-292)
*Bacillus subtilis* NA-6128 (IFO 14373, FERM BP-617)
*Bacillus subtilis* NA-7821 (IFO 14368, FERM BP-618)
*Bacillus subtilis* ATCC 19221
*Bacillus subtilis* ATCC 14662
*Bacillus pumilus* (FERM P-2116)
*Bacillus pumilus* (FERM P-4832)
*Bacillus pumilus* (FERM P-4829)
*Bacillus licheniformis* IFO 12485
When used to transform a *Bacillus* strain, a novel DNA of the present invention can be used in the intact form of a plasmid; however, it is normally preferable that either a linear DNA or a DNA of the present invention be used after being digested from the plasmid.

The transformation of a *Bacillus* strain with a linear DNA fragment can be carried out using a published method [C. Anagnostopouls and J. Spizizen; J. Bacteriol., *81* 741 (1961)]; the resulting transformant can be picked up from the colony grown on the selective medium using a selection marker. For example, when a drug resistance gene has been inserted as a selection marker, the resulting transformant can be picked up from the medium for selection containing the relevant drug. Judgment can be made of whether or not the transformant thus obtained has become an IMP dehydrogenase deficient strain as desired for the present invention by examining its growth on each of a minimum medium containing the substances essential to the growth of the recipient and a xanthine-supplemented medium prepared from said minimum medium.

It can be confirmed that said transformant is a novel microbe by cleaving the chromosomal DNA of said transformant with a suitable restriction enzyme, transferring the fragments thus obtained onto nitrocellulose filters after agarose gel electrophoresis and alkali denaturation, separately labeling the DNA fragment used for the insertion, for example, a DNA fragment containing a drug resistance gene, with radioactivity, and conducting Southern hydridization using this DNA fragment as a probe. That is it, can be easily judged that said transformant is a desired microbe of the present invention based on the fact that a fragment which hybridizes to the probe is present in the chromosomal DNA of said transformant while no fragment hybridizable to the probe is present in the chromosomal DNA of the recipient.

It can be confirmed that said DNA fragment has been inserted onto the IMP dehydrogenase gene, based on the fact that when Southern hybridization is conducted using as a probe IMP dehydrogenase isolated from the donor, the fragment which hybridizes to said probe of the chromosomal DNA of the transformant has become larger than that of the recipient by the length of the inserted DNA fragment. Similarly, the transformant obtained by the use of the DNA deleting a part or the whole of IMP dehydrogenase gene can be confirmed from the comparison with the length of DNA fragments when hybridized.

The above transformant, whose chromosomal DNA has an IMP dehydrogenase gene onto which a marker gene is inserted, has become drug-resistant due to marker gene expression when the marker gene is, for example, a drug resistance gene, and it has become IMP dehydrogenase deficient and xanthine-requiring due to marker gene insertion; however, the transformant is not different from the pre-transformation recipient in any other bacteriological characteristics.

Said transformant is also characterized in that the back mutation rate for xanthine requirement is at or below the detection limit.

The back mutation rate can be shown in the ratio of the growth colony count on an agar plate medium containing no xanthine to the number of bacterial cells applied over said aga plate medium. However, the back mutation rate of said transformant is extremely low; no revertant occurs even when the transformant is subjected to subculture or mutagenic agent treatment.

As examples of such transformants of the present invention, mention may be made of *Bacillus subtilis* BM 1041 (IFO 14560; FERM BP-1243, deposited on December 25, 1986) *Bacillus subtilis* NA-6141 (IFO 14561; FERM BP-1244 deposited on December 25, 1986) *Bacillus subtilis* BM-1403 (IFO 14655; FERM BP-1501, deposited on October 7, 1987), as in the examples shown below.

By selecting suitable marker genes and recipients, various transformants can certainly be easily constructed as well in accordance with the method described in the present specification.

Next, for the production of inosine using a transformant obtained by the present invention, a method similar to the conventional cultivation method for inosine-producing microorganisms except that a source of xanthine, a substance required by said transformant, is added to the medium.

That is, for the medium, media containing a carbon source, a nitrogen source and metal ions and where necessary nutrients such as amino acids, nucleic acids and vitamins. Carbon sources which can be used include glucose, sucrose, maltose, starch, saccharified-starch solution and molasses. Nitrogen sources which can be used, either singly or in combination, include inorganic nitrogen sources such as ammonium salts of sulfuric acid, nitric acid, hydrochloric acid and carbonic acid, ammonia gas and aqueous ammonia as well as organic nitrogen sources such as peptone, corn steep liquor, soybean meal, yeats and urea. As to other nutrients, various minerals, amino acids, vitamins and the like essential to bacterial growth are used either singly or in combination after proper selection. Substances which can be used as xanthine sources include not only xanthine, xanthosine, xanthylic acid and substitutes for them such as guanine, guanosine, guanylic acid and nucleic acid but also microbial cells containing them and extracts from such microbial cells. Substances which can be used as adenine sources include not only adenine, adenosine, adenylic acid and nucleic acid but also microbial cells containing them and extracts from such microbial cells.

In addition, antifoam agents or surfactants such as silicon oil and polyalkylene glycol ether may be added to the medium as occasion needs.

The cultivation is normally carried out under aerobic conditions such as shake culture and aerobic submerged culture. It is normally preferable that the pH of the medium be in a range of 4 to 9. In cases where a pH fluctuation is noted during cultivation, sulfuric acid, calcium carbonate, sodium hydroxide, ammonia gas, aqueous ammonia or the like may be properly added to adjust the pH to a preferable range. As to cultivation temperature, a temperature suitable for both the growth of the used microbe and inosine accumulation is selected out of a range of 20 to 40°C. Cultivation is carried out until the amount of accumulated inosine actually reaches its maximum, but the purpose normally is achieved by 24 to 144 hours of cultivation.

For separating and harvesting inosine from the culture, already published ordinary means of purification such as precipitation and chromatography techniques using ion exchange resin or activated charcoal.

By the present invention inosine, a starting material for 5ʹ-inosinic acid, an important seasoning substance, can be industrially favorably produced at high efficiency. That is, novel IMP dehydrogenase deficient bacterial strains of high inosine productivity can be obtained by transforming *Bacillus* strains capable of producing at least one of inosine, xanthosine and guanosine as recipients using the DNAs of the present invention. The inosine producing strains thus obtained are both high in inosine accumulation and highly stable because they have no back mutation; they are therefore very favorable also from the point of view of production control.

### Brief Description of the Drawings

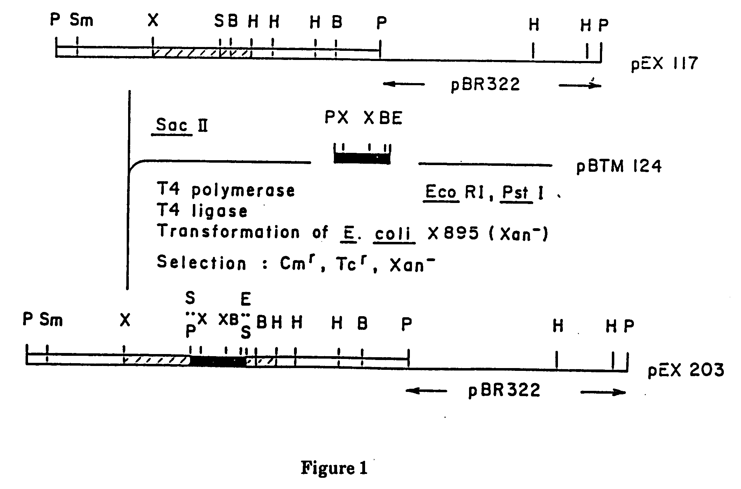

Figure 1 shows the method of preparing pEX 203 as obtained in Examples. In the figure, P, Sm, X, S, B, H and E respectively represent the restriction sites of the restriction enzymes *Pst* I*, Sma* I*, Xba* I*, Sac* II*, Bam* HI*, Hin* cII *and Eco* RI 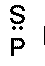 means that the cohesive end of *Sac* II and that of *Pst* I were ligated together after being each converted to a blunt end with T4 DNA polymerase. The shaded portion in the figure represents the IMP dehydrogenase gene region, and the heavy line represents the chloramphenicol acetyltransferase gene region.

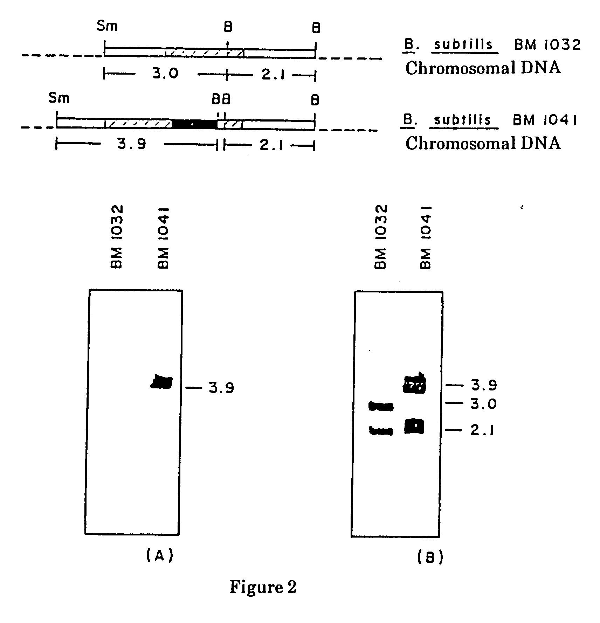

The upper diagrams of Figure 2 respectively schematize the structures of *Bacillus subtilis* BM 1032 chromosomal DNA and *Bacillus subtilis* BM 1041 chromosomal DNA; in the diagrams, Sm, B, the figures, the shaded portion and the heavy line respectively represent *Sma* I restriction site, *Bgl* II restriction site, the numbers of base pairs (kilobase pairs), the IMP dehydrogenase gene region and the chloramphenicol acetyltransferase gene region. The lower diagrams A and B of Figure 2 respectively show hybridization of the *Sma* I-*Bgl* II double digested products of the chromosomal DNA of *Bacillus subtilis* BM 1032 and *Bacillus subtilis* BM 1041 with the ³²P-labeled pBTM 124 chloramphenicol acetyltransferase gene DNA, and hybridization of the *Sma* I-*Bgl* II double digested products of the chromosomal DNAs of *Bacillus subtilis* BM 1032 and *Bacillus subtilis* BM 1041 with the ³²P-labeled pEX 117 IMP dehydrogenase gene region.

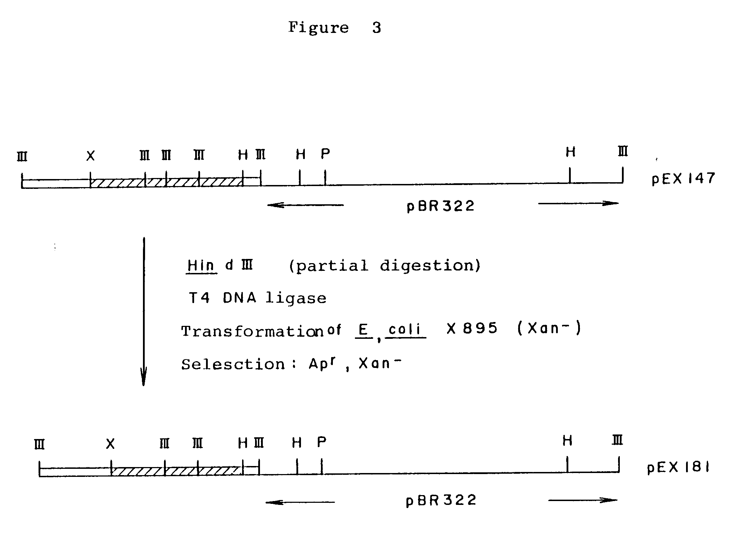

Figure 3 shows the method of preparing p EX 181 as described in Example 5. In the figure, III, X and P respectively represent *Hind* III, *Xba*I and *Pst* I.

The present invention is hereinafter described in more detail with some reference examples and embodiment examples, but the present invention certainly is not limited to these cases. Unless otherwise specified, the reactions using restriction enzymes (all were manufactured by Nippon Gene K.K.) or modification enzymes described in Reference Examples or Examples below were carried out under the conditions specified by respective enzyme manufacturers.

### Reference Example 1

### Isolation of the plasmid pEX 117 containing a Bacillus subtilis IMP dehydrogenase gene:

From the transformant *Esherichia coli* TEX 117 (Japanese Published unxamined Patent Application No. 156388/1985) carrying the recombinant plasmid pEX 117 containing an IMP dehydrogenase gene region derived from *Bacillus subtilis* strain NA-6012 (IFO 14190, FERM BP-292), the said plasmid was extracted in accordance with the method described in "Molecular Cloning", pp. 86 to 93. That is, *Esherichia coli* TEX 117 was inoculated to an LB medium (10g/ℓ Bacto Trypton, 5 g/ℓ yeast extract, 5 g/ℓ sodium chloride), and chloramphenicol was added to a concentration of 140 µg/mℓ for plasmid amplification, followed by cultivation overnight, then bacterial cells were harvested and washed. The washed cells were lysed by adding lysozyme and further adding a 0.2N sodium hydroxide solution containing 1% sodium lauryl sulfate, followed by the addition of 5M potassium acetate, then a supernatant containing the plasmid was obtained via centrifugation. To the resulting supernatant a 0.6 volume of isopropanol was added to precipitate the plasmid DNA, which was then washed with ethanol and dissolved in a TE buffer solution (10mM tris-hydrochloride buffer solution, 1mM EDTA, pH 8.0). To the resulting solution cesium chloride was added to a specific gravity of 1.60, and ethidium bromide was added to a final concentration of 600 µg/mℓ. Using an ultracentrifuge (rotor: V₆₅ Ti), centrifugation was carried out at 20°C and 50,000 rpm for 12 hours; the plasmid band detectable by ultraviolet was taken, and ethidium bromide was extracted and removed with *n*-butanol, followed by dialysis to a TE buffer solution; as a result, the recombinant plasmid pEX117 in an amount corresponding to about 200 µg was obtained from 300 mℓ of the culture broth. pEX 117 was cleaved with various restriction enzymes, and the restriction map shown in Figure 1 was prepared from the agarose gel electrophoresis patterns of the plasmid fragments thus obtained, based on the molecular weight of the lambda phage *Hin* dIII digested DNA. pEX 117 is a recombinant plasmid wherein an about 6.4 kilobase pairs of DNA fragment has been inserted at the *Pst* I site of pBR 322.

3µg of pEX117 was partially digested with the restriction enzyme *Hin*d III. Separately, pBR322 was completely digested with *Hind* III. The two digests were mixed and ligated together by the method described above. This DNA solution was used for transformation of *Escherichia coli* X-895 by the procedure described above. Tetracycline resistant strains were thus obtained and a transformant which was no longer xanthine-requiring, i.e. *Escherichia coli* TEX-147, was obtained from among them. About 220 µg of recombinant plasmid was obtained from said transformant by plasmid extraction conducted by the method described above. The plasmid was named pEX147. The restriction enzyme cleavage map of pEX147 is shown in Fig. 3. pEX147 is a recombinant plasmid derived from pBR322 by insertion of 2.9 kilo base pairs DNA fragment at the *Hin*d III site thereof.

### Reference Example 2

### Isolation of the plasmid pBTM 124 containing a chloramphenicol acetyltransferase gene:

The recombinant plasmid pBTM 124 containing a *Bacillus pumilus*-derived chloramphenicol acetyltransferase gene was prepared in accordance with the method of Williams et al. [J. Bacteriol., *146*, 1162 (1981)] as follows:
A DNA was prepared from *Bacillus pumilus* NCIB 8600 (IFO 12089, listed in the LIST OF CULTURES, 7th edition, issued by the INSTITUTE FOR FERMENTATION, OSAKA). The DNA (6.5 µg) was cleaved by reacting it with 40 units of the restriction enzyme *Eco* RI at 37°C for 1 hour, followed by heating at 68°C for 15 minutes and ethanol precipitation. Separately, the plasmid pUB 110 (2.0 µg) was cleaved by reacting it with 20 units of the restriction enzyme *Eco* RI at 37°C for 1 hour, followed by heating at 68°C for 15 minutes and ethanol precipitation.

Both resulting precipitates were dissolved in water and mixed together; a reaction liquid (100 µℓ) prepared by adding 60 nmole ATP, 10 units of T4 DNA ligase and a ligase buffer solution to the mixture thus obtained was incubated at 11°C for 30 hours, followed by ethanol precipitation. The resulting precipitate was dissolved in a TE buffer solution (50 µℓ). Using 25 µℓ of the resulting solution, *Bacillus subtilis* MI-114 [Gene, 24, 255 (1983)] was transformed to obtain a chloramphenicol-resistant transformant. From the chloramphenicol-resistant transformant thus obtained, a plasmid was prepared, which was named pBTM 124. pBTM 124 is a recombinant plasmid wherein an about 2.1 kilobase pairs of DNA fragment containing a chloramphenicol acetyltransferase gene has been inserted at the *Eco* RI site of pUB110.

### Example 1

30µg of pBTM 124 was double digested by reacting it with the restriction enzymes *Eco* RI and *Pst* I at 37°C for 1 hour, followed by agarose gel electrophoresis, and the agarose gel corresponding to the electrophoresis distance of a 1.1 kilobase pairs of DNA was cut off. Using an Unidirectional Electroeluter (manufactured by International Biotechnologies, Inc.), the relevant DNA was extracted from the above agarose gel fragment, followed by ethanol precipitation; the resulting precipitate was dissolved in a TE buffer solution (10 µℓ). Separately, 3 µg of pEX 117 was cleaved by reacting it with the restriction enzyme *Sac* II at 37°C for 1 hour, followed by heating at 65°C for 15 minutes and ethanol precipitation; the resulting precipitate was dissolved in a TE buffer solution (10 µℓ). Both resulting solutions were next mixed together; the resulting mixture was reacted with 2 nmole of dNTP and 5 units of T4 DNA polymerase (manufactured by Takara Shuzo Co., Ltd.) at 37°C in a TA buffer solution (33mM tris-acetate pH 7.9, 66mM potassium acetate, 10mM magnesium acetate, 0.5mM dithiothreitol, 0.01% bovine serum albumin) for 20 minutes, followed by heating at 65°C for 15 minutes, then phenol extraction and then ethanol precipitation were carried out. The resulting precipitate was dissolved in a TE buffer solution; 30 µℓ of the reaction mixture prepared by adding 66 nmole of ATP, 200 units of T4 DNA ligase (manufactured by Takara Shuzo Co., Ltd.) and a ligase buffer solution to the solution thus obtained was incubated at 16°C for one day. Using 5 µℓ of this reaction mixture, competent cells of *Esherichia coli* strain X895 (IFO 14308, FERM BP-614) were transformed to obtain the chloramphenicol-resistant transformant *Esherichia coli* X895 (pEX 203). This transformation was conducted in accordance with the method of Cohen et al. [Pro. Natl. Acad. Sci. USA, *69*, 2110 (1972)].

A plasmid was next extracted from the transformant *Esherichia coli* X895 (pEX 203) in accordance with the above mentioned method of Reference Example 1 and named pEX 203. pEX 203 was cleaved with various restriction enzymes, and its reaction map was prepared from the agarose gel electrophoresis pattern, based on the molecular weight of the lamba phage *Hin* dIII digested product (refer to Figure 1).

100 µg of pEX 203 was next cleaved with the restriction enzyme *Pst* I, followed by agarose gel electrophoresis; the agarose gel fragment corresponding to the electrophoresis distance of a 7.5 kilobase pairs of DNA was cut off; using the above-mentioned electroeluter, a DNA was extracted to isolate about 35 µg of DNA. Based on the restriction patterns, it was confirmed that the said DNA is a 7.5 kilobase pairs of DNA fragment inserted at the *Pst* I site of pBR 322, and it has a structure wherein a *Bacillus pumilus* NCIB 8600 chloramphenicol acetyltransferase gene has been inserted at the *Sac* II site on a *Bacillus subtilis* IMP dehydrogenase gene. The method of preparing pEX 203 and its restriction map are shown in Fig. 1.

### Example 2

### i) Derivation of a novel Bacillus strain having a chromosomal DNA of the structure wherein a chloramphenicol acetyltransferase gene has been inserted onto an IMP dehydrogenase gene:

Using conventional procedures of obtaining auxotrophs, the adenine-requiring strain *B. subtilis* BM 1032 (IFO 14559, FERM BP-1242) was obtained from *Bacillus subtilis* MI-114.

Competent cells of the said bacterium were next prepared in accordance with the method of C. Anagnostpoulos and J. Spizizen. To 1 mℓ of a suspension of the said competent cells the 7.5 kilobase pairs of DNA fragment obtained in Example 1 was added, followed by shaking at 37°C for 1 hour. The suspension was then applied onto an LB agar medium containing 10 µg/mℓ chloramphenicol and incubated at 37°C for one day; from the resulting colony was obtained the chloramphenicol-resistant transformant *Bacillus subtilis* BM 1041 (IFO 14560, FERM BP-1243). The said transformant was inoculated to an M-9 CATA medium (6 g/ℓ Na₂HPO₄, 3g/ℓ KH₂PO₄, 0.5 g/ℓ NaCl, 1 g/ℓ NH₄Cl, 1mM MgSO₄, 1mM CaCl₂, 2 g/ℓ glucose, 2 g/ℓ casamino acid, 50 mg/ℓ adenine, 50 mg/ℓ tryptophan, 15 g/ℓ agar, pH 7.2) and a medium prepared by further adding xanthine to the above medium, and its growth was examined; as a result, it was confirmed that the said chloramphenicol-resistant transformant has come to exhibit an xanthine requirement as well. Measurements were made of IMP dehydrogenase activity in accordance with the method of Magasanik (Method in Enzymology VI, 106 to 110), but the said enzyme activity was not detectable in this bacterium.

### ii) Evidence that a chloramphenicol acetyl transferase gene has been inserted into the chromosome:

*Bacillus subtilis* BM 1041 was inoculated onto an LB medium and cultured at 28°C overnight, then a chromosomal DNA was obtained from bacterial cells in 1 ℓ of the culture broth via the method of purification of chromosomal DNA with phenol [Biochem. Biophys. Acta, *72* 619 (1963)]. 10 µg of the chromosomal DNA thus obtained was double digested with the restriction enzymes *Bgl* II and *Sma* I, followed by agarose gel electrophoresis, then the resulting cuttings were transferred onto nitrocellulose filters in accordance with the above-mentioned method described in "Molecular Cloning", pp. 382 to 386. The *Bgl* II-*Sma* I digested fragments of the chromosomal DNA of *Bacillus subtilis* BM-1032 were also transferred to nitrocellulose filters at the same time. Separately, a 1.1 kilobase pairs of DNA fragment was extracted and isolated from pBTM 124 by the method described in Example 1; this DNA fragment was then labeled with ³²P-CTP and used as a probe. The preparation of the said probe was carried out using the Nick Translation Kit-2 (manufactured by Nippon Gene) in accordance with the protocol specified by the manufacturer.

Southern hybridization was then conducted in accordance with the method described in "Molecular Cloning", pp. 387 to 389; as a result, a hybridization band appeared at the position corresponding to the phoresis distance of the 3.9 kilobase pairs of the chromosomal DNA of *Bacillus subtilis* BM-1041. No hybridization was noted to the chromosomal DNA of *Bacillus subtilis* BM 1032.

30 µg of pEX 117 was then double digested with *Xba* I and *Hin* cII, followed by agarose gel electrophoresis, then a small piece of agarose gel was cut off from the portion corresponding to the phoresis distance of a 1.85 kilobase pairs of DNA, and a DNA was extracted from the agarose gel fragment and labeled by nick translation as above. Using this labeled DNA as a probe, Southern hybridization was carried out in the same manner as above; as a result, hybridization bands appeared at the positions corresponding to the phoresis distances of a 2.1 kilobase pairs of DNA and a 3.0 kilobase pairs of DNA in the case of hybridization to the chromosomal DNA of *Bacillus subtilis* BM 1032 while hybridization bands appeared at the positions corresponding to the phoresis distances of a 2.1 kilobase pairs DNA and a 3.9 kilobase pairs of DNA in the case of hybridization to the chromosomal DNA of *Bacillus subtilis* DM 1041. Figure 2 shows these results.

### iii) Back mutation for xanthine requirement in Bacillus subtilis BM 1041:

A loopful of *Bacillus subtilis* BM 1041 was inoculated to 20 mℓ of a seed medium as described in Table 1 and subjected to shake cultivation at 37°C for 18 hours, then 1 mℓ of the culture broth was transferred to a main fermentation medium (20 mℓ) as described in Table 1 and subjected to shake cultivation at 37°C for 2 days. 1 mℓ of the resulting culture broth was transferred to a main fermentation medium as described in Table 1 and subjected to shake cultivation at 37°C for 2 days. After repeating transfer 5 times as above, the viable bacterial cell count in the culture broth was examined; as a result, the viable cell count per 1 mℓ of the culture broth was 1 X 10⁹, all of which had a xanthine requirement, and no revertant was noted.

Separately, *Bacillus subtilis* strain RN 63 (IFO 14307, FERM BP-613), a xanthine-requiring strain induced via a conventional method of obtaining transformants, was subjected to the same procedure as above; as a result, the viable cell count per 1 mℓ of the culture broth was 1 X 10⁹, 20% of which (2 X 10⁸) were revertants exhibiting no xanthine requirement.

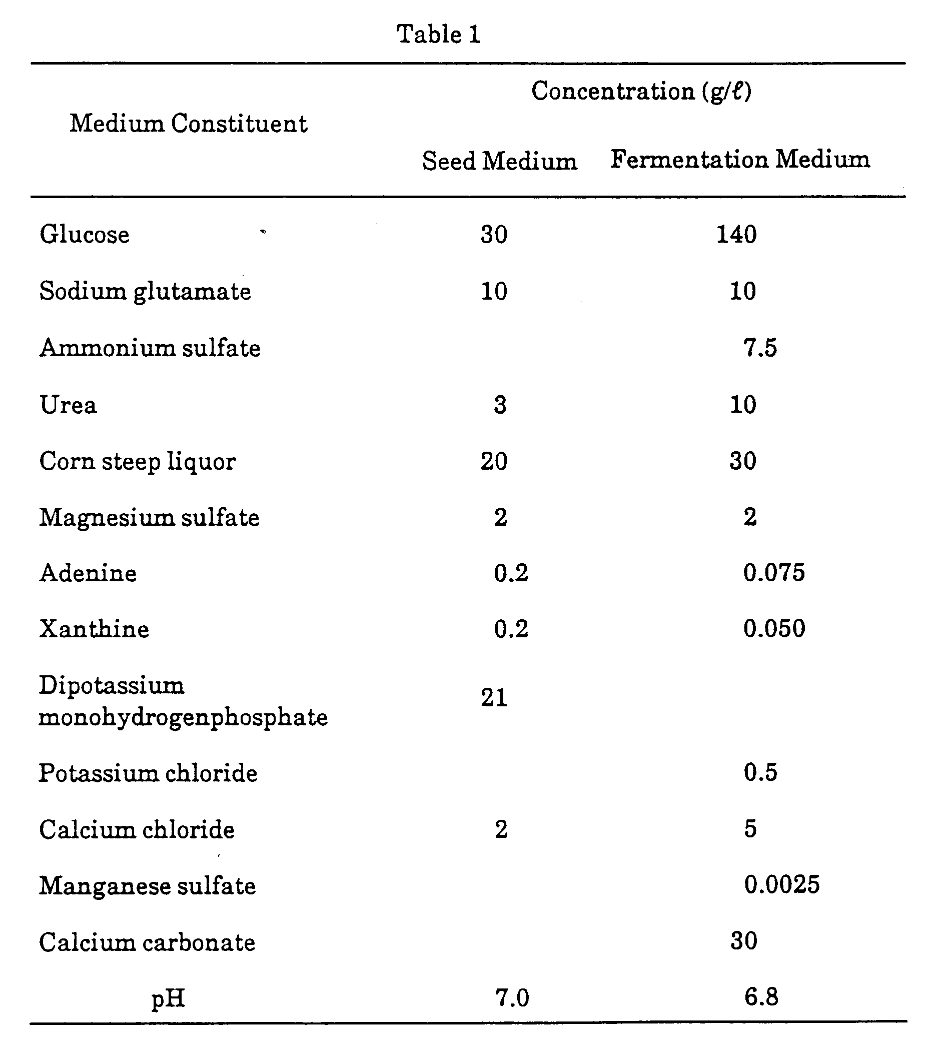

### iv) Inosine productivity of Bacillus subtilis BM 1041

A loopful of *Bacillus subtilis* strain BM 1041 was inoculated to a 1 ℓ capacity conical flask containing 125 mℓ of a seed medium as shown in Table 1 and subjected to shake cultivation at 37°C for 12 hours. The entire amount of the culture broth was transferred to a 5 ℓ capacity jar containing 2.5 ℓ of a main fermentation medium as shown in Table 1 and cultured at 37°C for 60 hours while both continuously feeding xanthine at a rate of 10 µg/mℓ·hr from 5 hours to 35 hours after the initiation of cultivation and adding 100 mℓ of a mixed solution containing 12.5% ammonium sulfate and 6.25% urea on the 24th hour following the initiation of main fermentative cultivation. After the completion of cultivation, the amounts of accumulated inosine and guanosine were examined via high performance liquid chromatography; as a result, inosine had accumulated in a ratio of 10.1 mg/mℓ. *Bacillus subtilis* strain BM 1032 was cultured under the same conditions as above; as a result, inosine accumulated in a ratio of only 5.2 mg/mℓ.

### Example 3

Competent cells of *Bacillus subtilis* strain NA-6128 (IFO 14373, FERM BP-617) were prepared in accordance with the method of Example 2. To the competent cells thus obtained the 7.5 kilobase pairs of DNA fragment obtained in Example 1 was added, after which they were shaken at 37°C for 1 hour for transformation; as a result, the chloramphenicol-resistant transformant *Bacillus subtilis* NA-6141 (IFO 14561, FERM BP-1244) was obtained from a colony which had grown on a LB medium containing 10 µg/mℓ chloramphenicol.

Based on whether or not growth had been noted on either of an M-9 CATA medium and an M-9 CATA medium further containing xanthine, the said transformant was found to have a xanthine requirement.

Southern hybridization was carried out by the same method as described in Example 2; as a result, no hybridization band was noted for the hybridization to the chromosomal DNA of *Bacillus subtilis* strain NA-6128 when the 1.1 kilobase pairs of DNA fragment of pBTM 124 was used as a probe, while hybridization was noted to the 3.9 kilobase pairs of *Sma* I-*Bgl* II fragment of the chromosomal DNA of *Bacillus subtilis* strain NA-6141. In addition, when the 1.85 kilobase pairs of *Xba* I-*Hin*cII fragment of pEX 117 was used as a probe, hybridization was noted to the DNA fragments respectively corresponding to 2.1 kilobase pairs and 3.0 kilobase pairs of *Sma* I-*Bgl* II trag of the chromosomal DNA of *Bacillus subtilis* strain NA-6128, while hybridization was noted to 2.1 kilobase pairs and 3.9 kilobase pairs of DNA fragments of the chromosomal DNA of *Bacillus subtilis* strain NA-6141.

### Example 4

A loopful of *Bacillus subtilis* strain NA-6141 was inoculated into a 1 ℓ capacity of conical flask containing 125 mℓ of a seed medium as shown in Table 1 and subjected to shake culture at 37°C for 12 hours. The whole amount of the resulting culture broth was transplanted to a 5 ℓ capacity of jar containing 2.5 ℓ of a main fermentation medium as shown in Table 1 and cultured at 37°C for 86 hours while both continuously feeding xanthine at a ratio of 10 µg/mℓ·hr from 5 hours to 35 hours after the initiation of cultivation and thereafter at a rate of 5 µg/mℓ·hr until the completion of cultivation and adding 100 mℓ of a mixed solution containing 12.5% ammonium sulfate and 6.25% urea on the 24th and 48th hours following the initiations of main fermentative cultivation. The amounts of inosine and guanosine which had accumulated in the culture broth were determined via high performance liquid chromatography; as a result, inosine was found to have accumulated at a ratio of 35.0 mg/mℓ. *Bacillus subtilis* strain NA-6128 was cultured under the same conditions as above; as a result, inosine accumulated at a ratio of only 19.8 mg/mℓ.

The viable cell count and xanthine-requiring revertant count in the above culture broth were examined; as a result, the viable cell count was 4 X 10⁸ per 1 mℓ of the culture broth, and no xanthine-requiring revertant was detected therein. The occurrence of adenine-requiring revertants was also examined for reference; as a result, such revertants were present in a ratio of 4 X 10² per 1 mℓ of the above culture broth. From the comparison of back mutation rate, the effect of the present invention is obvious.

### Example 5

i) 3 µg of pEX147 DNA was partially digested with the restriction enzyme *Hin*d III (1 unit), and the enzyme was inactivated by heating at 65°C for 10 minutes, and then said DNA was precipitated by adding cold ethanol to the reaction mixture. The precipitated DNA was collected by centrifugation, dissolved in 20 µl of a TE buffer solution and religated with T4 DNA ligase.
   The ligation mixture was used to transform *Escherichia coli* X895 in accordance with the method described in Example 1, and the cell suspension was plated onto an M-9 CATA agar plate medium containing ampicillin (25 µg/ml) and xanthine. After overnight incubation at 37°C, colonies formed on the agar plate medium were transferred onto an M-9 CATA agar medium and incubation was continued for farther 24 hrs. Growth on each agar plate medium was examined and ampicillin resistant- and xanthine requiring transformant named strain TEX181 was selected. A plasmid was isolated from the cells of strain TEX 181 in accordance with the method described in Reference Example 1, and was named pEX 181. Construction and restriction map of pEX 181 are shown in Figure 3.
   As is obvious from Figure 3, pEX 181 is a plasmid with deletion of *Hin*d III fragment which is about 0.25 kilo base pairs and located nearly at the centre of the inserted DNA fragment containing the IMP dehydrogenase gene region of pEX 147, and has no complementarity of xanthine requirement of *Escherichia coli* X895.
ii) *Pst* I digested DNA of pEX 181 (5 µg) was added to the competent cell suspension of *Bacillus subtilis* BM1031 and it was incubated at 37°C for 1 hr. Then the suspension was diluted with sterile water and plated onto M-9 CATA agar plate medium containing xanthine. After overnight incubation at 37°C, colonies formed on the plate medium were transferred onto an M-9 CATA agar medium and incubated for farther 24 hrs. Based on growth on a couple of media mentioned above, a xanthine requiring strain was selected and named *Bacillus subtilis* BM-1043 (IFO 14655, FERM BP-1501) IMP dehydrogenase activity of this strain was not detected.
iii) A loopful of *Bacillus subtilis* BM-1043 was inoculated into a LB medium and incubation was carried out at 28°C overnight. From cells harvested from the culture broth, chromosomal DNA was purified by the extraction method using phenol. 10 µg portion of chromosomal DNA thus obtained was digested with *Bgl* II and *Sma* I digested fragments of the chromosomal DNA of *Bacillus subtilis* BM1032 were also transferred on to nitro cellulose filter in the same manner as above. Southern hybridization was next carried out using 32p-labeled *Xba* I-*Hinc* II fragment, which is prepared in Example 2-ii, as probe. As the result, hybridization bands were observed at the position corresponding to the phoresis distance of 2.1 and 3.0 kilo base pairs in the case *Bacillus subtilis* BM-1043 chromosomal DNA.
iv) Back mutation of xanthine requirement of *Bacillus subtilis* BM-1043 was examined according to the procedure described in Example 2-iii), and no revertant was ovserved.
v) Production of inosine by *Bacillus subtilis* BM-1043 was examined by the method of Example 2-iv), and it was found that 9.8 mg/ml of inocine was accumulated in the culture broth.

## Claims

1. A method of producing inosine which comprises cultivating in a medium an IMP dehydrogenase deficient Bacillus subtilis strain transformed with a DNA encoding an inactivated IMP dehydrogenase, wherein said DNA comprises a DNA of an IMP dehydrogenase gene of Bacillus subtilis strain which has at least 1.85 kilobase pairs of the Xba-I-Hin CII fragment, and a selection marker gene, and harvesting inosine so produced in the culture medium.

2. The method according to claim 1, wherein the selection markers gene is one or more members selected from the group consisting of drug resistance genes, amino acid biosynthesis genes, nucleic acid biosynthesis genes and vitamin biosynthesis genes.

3. The method according to claim 2, wherein the drug resistance gene is a chloramphenicol acetyltransferase gene.

4. The method according to any of the preceding claims, wherein the IMP dehydrogenase deficient Bacillus subtilis strain in capable of producing inosine and exhibits no back mutation for xanthine requirement.

5. A method according to any of the preceding claims, wherein the IMP dehydrogenase deficient Bacillus subtilis strain is selected from the group consisting of Bacillus subtilis NA-6141 (FERM BP-1244), and Bacillus subtilis BM 1041 (FERM BP-1243).

## Patentansprüche

1. Verfahren zur Herstellung von Inosin, umfassend das Kultivieren in einem Medium eines IMP-Dehydrogenase-Mangelstammes von Bacillus subtilis, der mit DNA transformiert ist, die eine inaktivierte IMP-Dehydrogenase codiert, worin die DNA eine DNA eines IMP-Dehydrogenase-Gens des Bacillus subtilis Stammes umfaßt, der wenigstens 1,85 Kilobasenpaare des Xba-I-Hin CII Fragments hat und ein Selektions-Marker-Gen und das Ernten des so in dem Kulturmedium hergestellten Inosins.

2. Verfahren gemäß Anspruch 1, worin das Selektions-Marker-Gen ein oder mehr Glieder sind, die aus der Gruppe, die aus Arzneimittelresistenz-Genen, Aminosäurebiosynthese-Genen, Nucleinsäurebiosynthese-Genen und Vitaminbiosynthese-Genen besteht, ausgewählt sind.

3. Verfahren gemäß Anspruch 2, worin das Arzneimittelresistenz-Gen ein Chloramphenicol-Acetyltransferase-Gen ist.

4. Verfahren gemäß irgendeinem der vorangehenden Ansprüche, worin der IMP-Dehydrogenase Mangelstamm von Bacillus subtilis befähigt ist, Inosin zu bilden und keine Rückmutation bei Xanthinbedarf zeigt.

5. Verfahren gemäß irgendeinem der vorangehenden Ansprüche, worin der IMP-Dehydrogenase Mangelstamm von Bacillus subtilis aus der Gruppe, die aus Bacillus subtilis NA-6141 (FERM BP-1244) und Bacillus subtilis BM 1041 (FERM BP-1243) besteht, ausgewählt ist.

## Revendications

1. Procédé de préparation de l'inosine, selon lequel on cultive dans un milieu une souche de Bacillus subtilis déficiente en déshydrogénase IMP, transformée par un ADN codant pour une déshydrogénoase IMP inactivée, caractérisé en ce que ledit ADN comprend un ADN d'un gène de déshydrogénase IMP d'une souche de Bacillus subtilis qui comporte au moins 1,85 kilobase paires du fragment Xba-I-Hin CII, et un gène de marqueur de sélection, et en ce que l'on récolte l'inosine ainsi produite dans le milieu de culture.

2. Procédé selon la revendication 1, caractérisé en ce que le gène de marqueur de sélection est un ou plusieurs membres choisis du groupe consistant en gènes de résistance à un médicament, gènes de biosynthèse d'acide aminé, gènes de biosynthèse d'acide nucléique et gènes de biosynthèse de vitamine.

3. Procédé selon la revendication 2, caractérisé en ce que le gène de résistance à un médicament est le gène d'acétyltransférase de chloramphénicol.

4. Procédé selon l'une quelconque des revendications précédentes, caractérisé en ce que la souche de Bacillus subtilis déficiente en déshydrogénase IMP est capable de produire de l'inosine et qui ne présente pas de mutation inverse pour une demande en xanthine.

5. Procédé selon l'une quelconque des revendications précédentes, caractérisé en ce que la souche de Bacillus subtilis déficiente en déshydrogénase IMP est choisie du groupe consistant en Bacillus subtilis NA-6141 (FERM BP-1244) et Bacillus subtilis BM 1041 (FERM BP-1243).
